# EUROPEAN PATENT SPECIFICATION

(11) **EP 1 843 710 B1**
(45) Date of publication and mention of the grant of the patent: **03.06.2015**
(21) Application number: 06720181.4
(22) Date of filing: 02.02.2006
(51) Int. Cl.: A61B 17/12, A61L 31/02, A61L 31/04, A61L 31/14

(54) **VASO-OCCLUSIVE DEVICES INCLUDING NON-BIODEGRADABLE BIOMATERIALS**
VASOOKKLUSIVE VORRICHTUNGEN MIT NICHT BIOLOGISCH ABBAUBAREN BIOMATERIALIEN
DISPOSITIFS VASO-OCCLUSIFS COMPORTANT DES BIOMATERIAUX NON BIODEGRADABLES

(30) Priority: 04.02.2005 US 51599
(43) Date of publication of application: 17.10.2007
(60) Divisional application: 11184252.2
(73) Proprietor: Stryker Corporation, Kalamazoo, MI 49002 (US); Stryker NV Operations Ltd, Dublin 2 (IE)
(72) Inventor: CARR-BRENDEL, Victoria, E., Pleasanton, CA 94566 (US)
(74) Representative: Lecomte, Didier
(86) International application number: PCT/US2006/003750
(87) International publication number: WO 2006/084076

(56) References cited:
- US-A1- 2002 193 823
- US-B1- 6 287 318
- US-B1- 6 440 166

## Description

### FIELD OF THE INVENTION

Compositions and methods for repair of aneurysms are described. In particular, vaso-occlusive devices are disclosed, as are methods of making and using these devices.

### BACKGROUND

An aneurysm is a dilation of a blood vessel that poses a risk to health from the potential for rupture, clotting, or dissecting. Rupture of an aneurysm in the brain causes stroke, and rupture of an aneurysm in the abdomen causes shock. Cerebral aneurysms are usually detected in patients as the result of a seizure or hemorrhage and can result in significant morbidity or mortality.

There are a variety of materials and devices which have been used for treatment of aneurysms, including platinum and stainless steel microcoils, polyvinyl alcohol sponges (Ivalone), and other mechanical devices. For example, vaso-occlusion devices are surgical implements or implants that are placed within the vasculature of the human body, typically via a catheter, either to block the flow of blood through a vessel making up that portion of the vasculature through the formation of an embolus or to form such an embolus within an aneurysm stemming from the vessel. One widely used vaso-occlusive device is a helical wire coil having windings which may be dimensioned to engage the walls of the vessels. (*See, e.g.,* U.S. Patent No. 4,994,069 to Ritchart et al.) Other less stiff helically coiled devices have been described, as well as those involving woven braids. *See, e.g.,* U.S. Patent No. 6,299,627.

U.S. Pat. No. 5,354,295 and its parent, U.S. Pat. No. 5,122,136, both to Guglielmi et al., describe an electrolytically detachable embolic device. Vaso-occlusive coils having little or no inherent secondary shape have also been described. For instance, co-owned U.S. Patent Numbers 5,690,666; 5,826,587; and 6,458,119 by Berenstein et al., describes coils having little or no shape after introduction into the vascular space. U.S. Patent No. 5,382,259 describes non-expanding braids covering a primary coil structure.

Vaso-occlusive devices comprising one or more coatings have also been described. U.S. Patent Nos. 6,280,457 discloses vaso-occlusive devices that include biodegradable coatings.

United States Patent US 6,602,269 B2 discloses an embolic device according to the preamble of appended claim 1. United States Patent US 6,287,318 B1 discloses a device for occluding a space within a body. In particular, this device typically comprises a metallic core or core member and two polymeric junctions of differing thrombogenicity.

However, none of the above documents show a device as described herein including one or more non-biodegradable materials that limit the formation of scar tissue and resist long-term recanalization of an aneurysm.

### SUMMARY OF THE INVENTION

The present invention is defined by appended claim 1. Preferred embodiments are set forth in the dependent claims.

Any of the devices described herein may further comprise an inner member. In certain embodiments, the non-biodegradable material is attached to the inner member at one or more locations. In any of the devices described herein, the inner member may comprise a metal (*e.g*., nickel, titanium, platinum, gold, tungsten, indium and alloys or combinations thereof), stainless steel or a super-elastic metal alloy. The inner member may be, for example, be shaped as a helical coil. In any of the devices described herein, the inner member may further comprise a biodegradable material and/or a bioactive component.

Any of the devices described herein may further comprise a severable junction detachably which may be connected to a pusher element. The detachment junction can be positioned anywhere on the device, for example at one or both ends of the device. In certain embodiments, the severable junction(s) are, an electrolytically detachable assembly adapted to detach by imposition of a current; a mechanically detachable assembly adapted to detach by movement or pressure; a thermally detachable assembly adapted to detach by localized delivery of heat to the junction; a radiation detachable assembly adapted to detach by delivery of electromagnetic radiation to the junction or combinations thereof. The detachment junction(s) may be attached to inner member or non-biodegradable material.

These and other embodiments of the subject invention will readily occur to those of skill in the art in light of the disclosure herein.

### BRIEF DESCRIPTION OF THE FIGURES

FIG. 1 is a perspective view of an exemplary vaso-occlusive device as described herein made up of strands comprising non-degradable biomaterials.
FIG. 2 is a perspective view of an exemplary vaso-occlusive device as described herein made up of strands comprising non-degradable biomaterials in combination with a coil-shaped inner member. Also shown is a detachment junction on the inner coil member.
FIG. 3 is a perspective view of an exemplary vaso-occlusive device as described herein as deployed within an aneurysm. The device assumes a random, three-dimensional configuration upon deployment.
FIG. 4 is a reproduction of an electron micrograph showing the pore structure of an exemplary non-degradable biomaterial attached to an inner platinum coil.
It is to be understood that the drawings depict only exemplary embodiments and are not to be considered limiting in scope.

### DESCRIPTION OF THE INVENTION

Occlusive (*e*.*g*., embolic) compositions are described. The compositions described herein find use in vascular and neurovascular indications and are particularly useful in treating aneurysms, for example small-diameter, curved or otherwise difficult to access vasculature, for example aneurysms, such as cerebral aneurysms.

It must be noted that, as used in this specification and the appended claims, the singular forms "a", "an", and "the" include plural referents unless the content clearly dictates otherwise. Thus, for example, reference to a device comprising "a strand" includes devices comprising of two or more elements.

The devices described herein comprise a non-biodegradable, porous component that aids arrest the foreign body response when implanted into a subject. By "non-biodegradable" or "non-degradable" is meant that a material that persists in the subject in essentially the same form as implanted for a period of time ranging from months to years. Similarly, by "biomaterial" is meant any substance or combination of substances synthetic or natural in origin, which can be used for any period of time, as a whole or as a part of a system that treats, augments, or replaces any tissue, organ, or function in a subject (*e*.*g*., mammal).

Aneurysms treated with biodegradable products that promote wound healing may result in the formation of scar tissue over time. Because scar tissue is an avascular, acellular mass made up mostly of extracellular matrix proteins, the interface between the scar tissue and healthy tissue is less robust than surrounding tissue and, as such, less resistant to long-term recanalization. The vaso-occlusive devices described herein comprise materials that are capable of arresting wound healing in a state of a modified foreign body response (also referred to as material microarchitecture-driven neovascularization) so that the resulting tissue response does not evolve into scar and, as such, is adjacent (continuous) with the healthy tissue and is more resistant to sheer, flow and recanalization. Notably, there is no demarcation between the response to the foreign body and the native tissue -- they are cocontinuous.

During a typical course of wound healing, neutrophils are the predominant cell type at the site of injury within the first 24-48 hours, killing and phagocytosing bacteria and/or cellular debris. After approximately 48 hours, macrophages become the predominant cell type, further removing cellular and foreign, debris from the wounded area. Within three to four days, fibroblasts migrate out of the surrounding connective tissue (e.g., intima) into the wound area and begin to synthesize collagen, which quickly fills the wound space, forming a complex tertiary structure consisting of both cells and extracellular matrix components. New blood vessels also begin to grow into the area at this time to supply oxygen and nutrients needed by the metabolically active fibroblasts and macrophages. However, in a typical course of wound healing, new vessel formation begins to regress in the second week, resulting formation of an avascular and acellular scar.

In contrast to a typical course of wound healing, in a modified foreign body response, material microarchitecture-driven neovascularization of new vessels does not regress and a multitude of vessels persist at the interface of the material and tissue. *See, e.g.,* Padera, et al. (1996) "Time Course of Membrane Microarchitecture-driven Neovascularization," 17 Biomaterials 277-84. The persistence of adjacent vascular structures maintains the integrity of the space filling in-growth with the adjacent preexisting vessel structures in a co-continuous fashion. Furthermore, the in-growth is then available as a substratum upon which endothelium can grow and persist.

Thus, the non-biodegradable vaso-occlusive devices as described herein preferably comprise one or more materials that favor an arrested foreign body response, which as described above is granular in nature, has new vessel formation and is typically intimately associated with the non-degradable material. Typically, the non-degradable component comprises one or more materials (*e*.*g*., polymers) having an average nominal pore size ranging from approximately 0.1 microns to about 20 microns (or any value therebetween), more preferably from about .5 microns to about 15 microns (or any value therebetween), and even more preferably from 0.8 microns to about 10 microns (or any value therebetween), using conventional methods for determination of pore size in the trade.

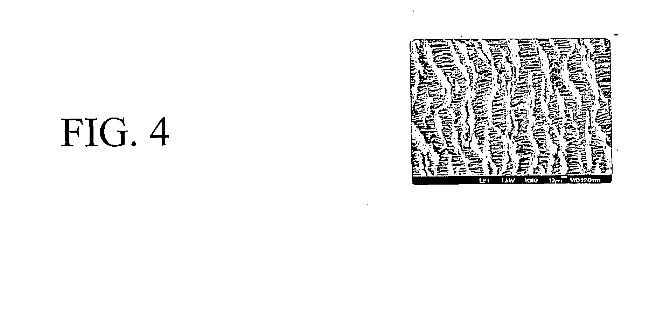

Suitable non-degradable materials include, cellulose acetate (pore size range from about 0.8 to about 8 microns); mixed esters cellulose (pore size range from about 1.2 microns to about 8 microns); PTFE/polyester (pore size range from about 1.0 microns to about 10 microns, see, also FIG. 4); and acrylic copolymer (pore size range from about 0.8 microns to about 10 microns). Although the use of some of these materials as chambers for living cells (*see*, *e.g.,* U.S. Patent Nos. 6,773,458; 5,964,804; 5,807,406) and as vascular grafts (*see, e.g.,* U.S. Patent No. 6,517,571) has been described, they have not been used as vaso-occlusive devices, likely because of their known antithrombogenic characteristics.

The non-degradable components of the devices may take a variety of three-dimensional configurations upon deployment into the subject, including structural elements which provide the three dimensional conformation may include fibers, strands, coils, globules, cones or rods of amorphous or uniform geometry which are smooth or rough. Generally, the three-dimensional configuration of the non-degradable component(s) have one dimension larger than the other two and the smaller dimensions preferably do not exceed five microns.

The non-degradable component can also be optionally used in combination with other vaso-occlusive devices, for example the GDC-type vaso-occlusive coils described above (*see, e.g.,* U.S. Patent Nos. 6,723,112; 6,663,607; 6,602,269; 6,544,163; 6,287,318; 6,280,457 and 5,749,894).

Depicted in the Figures are exemplary embodiments of the present invention. Although the optional inner member is depicted as a coil, it will be appreciated that this is for purposes of illustration only and that the inner member can be of other shapes, for example different shaped coils, stents, mandrels, wires, filters or the like.

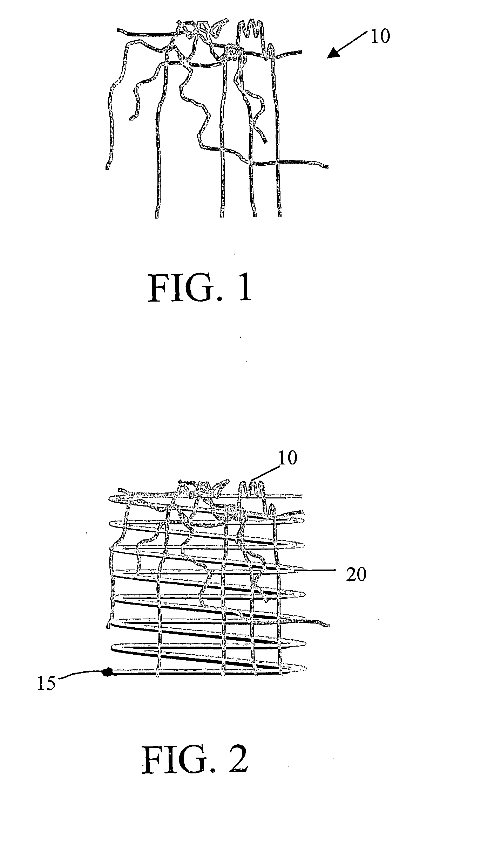

FIG. 1 depicts an exemplary embodiment of the inventive vaso-occlusive devices described herein. The device as a whole is generally designated (10) and is shown in a three-dimensional strand configuration.

FIG. 2 shows the exemplary embodiment of FIG. 1 in combination with GDC-type vaso-occlusive coil (20). As shown in FIG. 2, the strands of the non-degradable device may wind in and out of the turns making up the three-dimensional coil (*e.g*., one or more of the strands passes through the lumen of the coil. Alternatively, the non-degradable device may be placed over the coil such that the strands are all on the exterior surface of the coil. Further, the non-degradable device (10) can be permanently or temporarily attached in one or more locations to the inner member by any suitable attachment mechanism. Also shown is detachment junction (15) positioned on the proximal end of the coil (20).

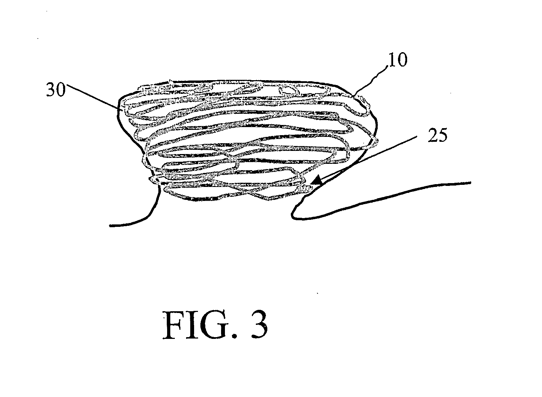

FIG. 3 shows an exemplary non-biodegradable device described herein, as deployed within an aneurysm (30). The device (10) assumes a three-dimensional configuration that substantially fills the aneurysm. Also shown in a small hook (25) at one end (e.g., proximal end) of the device, which may aid in attachment to the wall of aneurysm. As shown, the inner member (coil) can be removed after the non-biodegradable member (30) has been deployed into the aneurysm. Optionally, additional (*e.g*., bioactive) components may be inserted into the aneurysm, for example into the spaces surrounding the filament.

The non-degradable devices described herein may assume a variety of structures including, but not limited to, braids, coil, stents (e.g., self-expanding stents) and combinations of these. Preferably, the devices are deployed in a primary linear configuration and assume a three-dimensional configuration upon deployment into the target vessel. For example, the devices may form a coil configuration or may have a substantially random space-filling relaxed configuration (FIG. 3) upon deployment.

Like the non-degradable component, the optional inner member may assume a variety of structure and be comprised of a variety of materials. Thus, although depicted in the Figures as a coil (e.g., platinum coil), the inner member may be of a variety of shapes or configuration including, but not limited to, braids, wires, knits, woven structures, tubes (e.g., perforated or slotted tubes), injection-molded devices and the like. *See, e*.*g*., U.S. Patent No. 6,533,801 and International Patent Publication WO 02/096273. Thus, the optional inner member may be made of a variety of materials, including but not limited to metals, polymers and combinations thereof.

In certain embodiments, the inner member is a braided structure comprising one or more metals or metal alloys, for example, Platinum Group metals, especially platinum, rhodium, palladium, rhenium, as well as tungsten, gold, silver, tantalum, stainless steel and alloys of these metals. Preferably, the inner member comprises a material that maintains its shape despite being subjected to high stress, for example, "super-elastic alloys" such as nickel/titanium alloys (48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum). Particularly preferred are the alloys described in U.S. Pat. Nos. 3,174,851; 3,351,463; and 3,753,700. Especially preferred is the titanium/nickel alloy known as "nitinol." The inner member may also comprise a shape memory polymer such as those described in International Publication WO 03/51444.

In certain preferred embodiments, the inner member is a platinum coil. The inner member may also change shape upon release from the restraining member, for example change from a constrained linear form to a relaxed, three-dimensional configuration upon deployment.

As shown in FIG. 2, any of the devices described herein may further comprise a detachment junction (15), which is severable. The detachment junction (15) may be connected to a pusher element, such as a pusher wire. The detachment junction can be positioned anywhere on the device, for example at one or both ends of the optional inner member. In certain embodiments, the inner member may be removed after deployment, for example when the detachment junction is positioned at the proximal end of an inner member (FIG. 2).

The severable junction(s) may be detached in a variety of ways, for example using an electrolytically detachable assembly adapted to detach by imposition of a current; a mechanically detachable assembly adapted to detach by movement or pressure; a thermally detachable assembly adapted to detach by localized delivery of heat to the junction; a radiation detachable assembly adapted to detach by delivery of electromagnetic radiation to the junction or combinations thereof. Furthermore, the detachment mechanism may be hydraulic, for example the pusher wire may be cannulated, for example to allow for saline injection through the pusher wire to push off the coil.

The devices described herein may also comprise additional components, such as co-solvents, plasticizers, coalescing solvents, bioactive agents, antimicrobial agents, antithrombogenic agents (*e*.*g*., heparin), antibiotics, pigments, radiopacifiers and/or ion conductors which may be coated using any suitable method or may be incorporated into the element(s) during production. The bioactive materials can be coated onto the device (e.g., heparin) and/or can be placed in the vessel prior to, concurrently or after placement of one or more devices as described herein. For example, in embodiments in which the inner member is removed after deployment of the non-degradable device, one or more bioactive materials can be delivered to the vessel.

As noted elsewhere, the location of the device is preferably visible using fluoroscopy. A highly preferred method is to ensure that at least some of the elements (e.g., small pore non-degradable member and/or inner member) making up the device are provided with significant radio-visibility via the placement of a radio-opaque covering on these elements. A metallic coating of a metal having comparatively more visibility, during fluoroscopic use, than stainless steel is preferred. Such metals are well known but include gold and members of the Platinum Group described above.

One of more of the elements may also be secured to each other at one or more locations. For example, to the extent that various elements are thermoplastic, they may be melted or fused to other elements of the devices. Alternatively, they may be glued or otherwise fastened. Furthermore, the various elements may be secured to each other in one or more locations.

### METHODS OF USE

The devices described herein are often introduced into a selected site using the procedure outlined below. This procedure may be used in treating a variety of maladies. For instance in the treatment of an aneurysm, the aneurysm itself will be filled (partially or fully) with the compositions described herein.

Conventional catheter insertion and navigational techniques involving guidewires or flow-directed devices may be used to access the site with a catheter. The mechanism will be such as to be capable of being advanced entirely through the catheter to place vaso-occlusive device at the target site but yet with a sufficient portion of the distal end of the delivery mechanism protruding from the distal end of the catheter to enable detachment of the implantable vaso-occlusive device. For use in peripheral or neural surgeries, the delivery mechanism will normally be about 100-200 cm in length, more normally 130-180 cm in length. The diameter of the delivery mechanism is usually in the range of 0.25 to about 0.90 mm. Briefly, occlusive devices (and/or additional components) described herein are typically loaded into a carrier for introduction into the delivery catheter and introduced to the chosen site using the procedure outlined below. This procedure may be used in treating a variety of maladies. For instance, in treatment of an aneurysm, the aneurysm itself may be filled with the embolics (e.g. vaso-occlusive members and/or liquid embolics and bioactive materials) which cause formation of an emboli and, at some later time, is at least partially replaced by neovascularized collagenous material formed around the implanted vaso-occlusive devices.

A selected site is reached through the vascular system using a collection of specifically chosen catheters and/or guide wires. It is clear that should the site be in a remote site, e.g., in the brain, methods of reaching this site are somewhat limited. One widely accepted procedure is found in U.S. Patent No. 4,994,069 to Ritchart, et al. It utilizes a fine endovascular catheter such as is found in U.S. Patent No. 4,739,768, to Engelson. First of all, a large catheter is introduced through an entry site in the vasculature. Typically, this would be through a femoral artery in the groin. Other entry sites sometimes chosen are found in the neck and are in general well known by physicians who practice this type of medicine. Once the introducer is in place, a guiding catheter is then used to provide a safe passageway from the entry site to a region near the site to be treated. For instance, in treating a site in the human brain, a guiding catheter would be chosen which would extend from the entry site at the femoral artery, up through the large arteries extending to the heart, around the heart through the aortic arch, and downstream through one of the arteries extending from the upper side of the aorta. A guidewire and neurovascular catheter such as that described in the Engelson patent are then placed through the guiding catheter. Once the distal end of the catheter is positioned at the site, often by locating its distal end through the use of radiopaque marker material and fluoroscopy, the catheter is cleared. For instance, if a guidewire has been used to position the catheter, it is withdrawn from the catheter and then the assembly, for example including the vaso-occlusive device at the distal end, is advanced through the catheter.

Once the selected site has been reached, the vaso-occlusive device is extruded, for example by loading onto a pusher wire. Preferably, the vaso-occlusive device is loaded onto the pusher wire via a mechanically or electrolytically cleavable junction (e.g., a GDC-type junction that can be severed by application of heat, electrolysis, electrodynamic activation or other means). Additionally, the vaso-occlusive device can be designed to include multiple detachment points, as described in co-owned U.S. Patent No. 6,623,493 and 6,533,801 and International Patent publication WO 02/45596. They are held in place by gravity, shape, size, volume, magnetic field or combinations thereof.

As noted above, it will also be apparent that in certain embodiments, the inner member (20) is removeable after deployment into the vessel. For example, the device can be deployed into a vessel (aneurysm) as described herein. Once deployed, the non-degradable device (10) is detached from the inner member (20) while the inner member (20) remains attached to the pusher wire (25) and can be removed from the vessel.

It will also be apparent that the operator can remove or reposition (distally or proximally) the device. For instance, the operator may choose to insert a device as described herein, before detachment, move the pusher wire to place the device in the desired location.

## Claims

1. A vaso-occlusive device (10) comprising a porous, non-biodegradable material
comprising a polymer selected from the group consisting of cellulose acetate, mixed esters cellulose, PTFE/polyester, acrylic copolymers and combinations thereof;
**characterized in that**
the non-biodegradable material has a pore size of less than about 20 microns; and wherein
the device (10) is adapted to have a linear primary configuration prior to deployment and a secondary three-dimensional configuration after deployment.

2. The device of claim 1, further comprising an inner member (20).

3. The device of claim 2, wherein the non-biodegradable material is attached to the inner member (20) at one or more locations.

4. The device of claim 2 or claim 3, wherein the inner member (20) comprises a metal.

5. The device of claim 4, wherein the metal is selected from the group consisting of nickel, titanium, platinum, gold, tungsten, iridium and alloys or combinations thereof.

6. The device of claim 5, wherein the metal is nitinol or platinum.

7. The device of any of claims 2 to 6, wherein the inner member (20) comprises a coil (20).

8. The device of claim 7, wherein the inner member (20) comprises a coil (20) comprising a stainless steel or super-elastic metal alloy.

9. The device of any of claims 2 to 8, wherein the inner member (20) further comprises a biodegradable material.

10. The device of any of claims 2 to 9, wherein the inner member (20) further comprises a detachment junction (15).

11. The device of claim 10, wherein the detachment junction (15) comprises an electrolytically detachable end adapted to detach from a pusher by imposition of a current on the pusher.

12. The device of any of claims 1 to 11, further comprising an additional component.

13. The device of claim 12, wherein the additional component is bioactive.

## Patentansprüche

1. Vasookklusive Vorrichtung (10), umfassend ein poröses, nicht biologisch abbaubares Material, umfassend ein Polymer, ausgewählt aus der aus Celluloseacetat, Mischester-Cellulose, PTFE/Polyester, Acrylcopolymeren und Kombinationen davon bestehenden Gruppe;
**dadurch gekennzeichnet, dass**
das nicht biologisch abbaubare Material eine Porengröße von weniger als ungefähr 20 Mikron aufweist; und wobei
die Vorrichtung (10) dazu eingerichtet ist, vor dem Entfalten eine lineare primäre Konfiguration und nach dem Entfalten eine sekundäre dreidimensionale Konfiguration aufzuweisen.

2. Vorrichtung nach Anspruch 1, weiter ein inneres Element (20) umfassend.

3. Vorrichtung nach Anspruch 2, wobei das nicht biologisch abbaubare Material an einer oder mehreren Stellen an dem inneren Element (20) befestigt ist.

4. Vorrichtung nach Anspruch 2 oder 3, wobei das innere Element (20) ein Metall umfasst.

5. Vorrichtung nach Anspruch 4, wobei das Metall aus der aus Nickel, Titan, Platin, Gold, Wolfram, Iridium und Legierungen oder Kombinationen davon bestehenden Gruppe ausgewählt ist.

6. Vorrichtung nach Anspruch 5, wobei das Metall Nitinol oder Platin ist.

7. Vorrichtung nach einem der Ansprüche 2 bis 6, wobei das innere Element (20) eine Wendel (20) umfasst.

8. Vorrichtung nach Anspruch 7, wobei das innere Element (20) eine Wendel (20) umfasst, welche eine Edelstahl- oder superelastische Metalllegierung umfasst.

9. Vorrichtung nach einem der Ansprüche 2 bis 8, wobei das innere Element (20) weiter ein biologisch abbaubares Material umfasst.

10. Vorrichtung nach einem der Ansprüche 2 bis 9, wobei das innere Element (20) weiter eine Abtrennverbindung (15) umfasst.

11. Vorrichtung nach Anspruch 10, wobei die Abtrennverbindung (15) ein elektrolytisch abtrennbares Ende aufweist, das zur Abtrennung von einem Schubelement durch Anlegen einer Spannung an dem Schubelement eingerichtet ist.

12. Vorrichtung nach einem der Ansprüche 1 bis 11, weiter eine zusätzliche Komponente umfassend.

13. Vorrichtung nach Anspruch 12, wobei die zusätzliche Komponente bioaktiv ist.

## Revendications

1. Dispositif d'occlusion vasculaire (10) comprenant une matière poreuse non biodégradable comprenant un polymère choisi parmi le groupe constitué par l'acétate de cellulose, des esters cellulosiques mixtes, du PTFE/polyester, des copolymères acryliques et leurs combinaisons, **caractérisé en ce que** la matière non biodégradable possède une dimension de pores inférieure à environ 20 microns, et dans lequel le dispositif (10) est conçu pour posséder une configuration primaire de forme linéaire avant son déploiement et une configuration secondaire en trois dimensions après son déploiement.

2. Dispositif selon la revendication 1, comprenant en outre un membre interne (20).

3. Dispositif selon la revendication 2, dans lequel la matière non biodégradable est fixée au membre interne (20) à un ou plusieurs endroits.

4. Dispositif selon la revendication 2 ou 3, dans lequel le membre interne (20) comprend un métal.

5. Dispositif selon la revendication 4, dans lequel le métal est choisi parmi le groupe constitué par du nickel, du titane, du platine, de l'or, du tungstène, de l'iridium, ainsi que leurs alliages ou leurs combinaisons.

6. Dispositif selon la revendication 5, dans lequel le métal est du nitinol ou du platine.

7. Dispositif selon l'une quelconque des revendications 2 à 6, dans lequel le membre interne (20) comprend une spirale (20).

8. Dispositif selon la revendication 7, dans lequel le membre interne (20) comprend une spirale (20) comprenant un alliage à base d'acier inoxydable ou d'un métal superélastique.

9. Dispositif selon l'une quelconque des revendications 2 à 8, dans lequel le membre interne (20) comprend en outre une matière biodégradable.

10. Dispositif selon l'une quelconque des revendications 2 à 9, dans lequel le membre interne (20) comprend en outre une jonction de détachement (15).

11. Dispositif selon la revendication 10, dans lequel la jonction de détachement (15) comprend une extrémité apte à se détacher par voie électrolytique conçue pour se détacher d'un poussoir par application d'un courant sur le poussoir.

12. Dispositif selon l'une quelconque des revendications 1 à 11, comprenant en outre un composant supplémentaire.

13. Dispositif selon la revendication 12, dans lequel le composant supplémentaire est bioactif.
